**Europäisches Patentamt**

**European Patent Office**

**Office européen des brevets**

(19)

(11) Veröffentlichungsnummer : **0 448 000 B1**

(12)

# EUROPÄISCHE PATENTSCHRIFT

(45) Veröffentlichungstag der Patentschrift :
**25.05.94 Patentblatt 94/21**

(51) Int. Cl.$^5$ : **C07C 1/20, C07C 11/02**

(21) Anmeldenummer : **91104102.8**

(22) Anmeldetag : **16.03.91**

(54) **Verfahren zur Erzeugung von niederen Olefinen.**

(30) Priorität : **23.03.90 DE 4009459**

(43) Veröffentlichungstag der Anmeldung :
**25.09.91 Patentblatt 91/39**

(45) Bekanntmachung des Hinweises auf die
Patenterteilung :
**25.05.94 Patentblatt 94/21**

(84) Benannte Vertragsstaaten :
**BE DE ES FR GB IT NL**

(56) Entgegenhaltungen :
**EP-A- 0 369 364**
**DD-A-27 052 6**
**DE-A- 2 615 150**
**DE-A- 2 827 385**
**DE-A- 3 132 024**
**US-A- 4 767 886**
**KOKOTAILO UND MEIER, 1979, London, GB:**
**"Pentasil family of high silica crystalline ma-**
**terial", Special publication Nr. 33 of the Che-**
**mical Society, Seiten 133-139**

(73) Patentinhaber : **SÜD-CHEMIE AG**
**Lenbachplatz 6**
**D-80333 München (DE)**
Patentinhaber : **METALLGESELLSCHAFT AG**
**Postfach 10 15 01,**
**Reuterweg 14**
**D-60015 Frankfurt (DE)**

(72) Erfinder : **Schneider, Michael, Dipl.Chem. Dr.**
**Waldparkstr. 54 a**
**W-8012 Ottobrunn-Riemerling (DE)**
Erfinder : **Schmidt, Friedrich, Dipl.Chem. Prof.**
**Dr.**
**Lug ins Land 52**
**W-8200 Rosenheim (DE)**
Erfinder : **Burgfels, Götz, Dipl.Chem. Dr.**
**Angerweg 20**
**W-8201 Bad Feilnbach (DE)**
Erfinder : **Buchold, Henning, Dr.-Chem.**
**Erzberger Str. 3**
**W-6450 Hanau am Main (DE)**
Erfinder : **Möller, Friedrich-Wilhelm, Dr.-Chem.**
**Breslauer Ring 15**
**W-6382 Friedrichsdorf (DE)**

(74) Vertreter : **Patentanwälte Dipl. Ing. R.**
**Splanemann Dr. B. Reitzner Dipl. Ing. K.**
**Baronetzky et al**
**Tal 13**
**D-80331 München (DE)**

## Beschreibung

Die Erfindung betrifft ein Verfahren zur Erzeugung von niederen Olefinen aus Methanol durch Umsetzung eines Methanol- und/oder Dimethyletherdampf und Wasserdampf enthaltenden Reaktionsgemisches in einem Röhrenreaktor an einem indirekt gekühlten Katalysator auf der Basis von kristallinen Alumosilikaten vom Pentasil-Typ mit einem Si/Al-Atomverhältnis von mindestens 10.

Ein derartiges Verfahren ist aus der US-A-4 058 576 bekannt. Im einzelnen wird bei diesem Verfahren Methanol in einer ersten Stufe an einem sauren Katalysator, wie gamma-Aluminiumoxid, in einer exothermen Kondensationsreaktion mindestens teilweise in Dimethylether umgewandelt. Auf diese Weise kann ein Teil der Reaktionswärme der in der zweiten Stufe erfolgenden Umsetzung des Methanols zu niederen Olefinen abgebaut werden, da die bei der exothermen Umsetzung erzeugte Wärme bei Verwendung von Dimethylether als Ausgangsmaterial geringer ist als bei Verwendung von Methanol.

In der zweiten Stufe erfolgt die Umsetzung über einem kristallinen Zeolith vom Typ ZSM5. Es handelt sich hierbei um ein kristallines Alumosilikat vom Pentasil-Typ mit einem Si/Al-Atomverhältnis von mindestens 10.

Die Umsetzung in der zweiten Stufe erfolgt in einem Röhrenreaktor, wobei als niedere Olefine vorzugsweise solche mit drei oder mehr Kohlenstoffatomen ($C_{3+}$-Olefine) erhalten werden. Diese niederen Olefine werden dann an dem ZSM5-Katalysator unter bestimmten Betriebsbedingungen in Kohlenwasserstoffe im Leichtbenzin (Gasolin)-Siedebereich umgewandelt. Der Anteil der $C_{3+}$-Olefine und der Gasoline hängt von den Reaktionsbedingungen ab. Die Umsetzung wird jedoch bevorzugt bei erhöhtem Druck durchgeführt, damit das Reaktorvolumen besser ausgenutzt wird.

Die US-A-4 058 576 gibt keine Anregungen, wie der Propylenanteil im erhaltenen Olefingemisch erhöht werden kann. Propylen stellt einen wichtigen Rohstoff für die Gewinnung von Polypropylen dar, wobei das aus Methanol erhaltene Propylen gegenüber dem durch thermische Spaltung von Kohlenwasserstoffen erhaltenen Propylen vorzuziehen ist, da es praktisch frei von Schwefelverbindungen ist.

Aus der DE-A-36 04 636 ist ein Verfahren bekannt, nach dem Methanol oder Dimethylether in Olefine an einem dealuminierten Mordenit umgewandelt wird. Der Propylengehalt des erzeugten Kohlenwasserstoffgemisches liegt trotz der aufwendigen Rückführung noch unter 50 %. Es wurde offenbar nicht erkannt, daß durch Verwendung von Katalysatoren vom Pentasil-Typ und durch eine Verminderung des Gesamtdruckes der Propylenanteil erhöht werden kann.

Die DE-C-32 28 269 beschreibt ein Verfahren zur Konvertierung von Alkoholen und/oder aliphatischen Ethern zu ungesättigten Kohlenwasserstoffen unter Verwendung eines Katalysators, der ZnO und/oder CdO enthält und dessen Zeolith mit einem Sulfoniumjodid hergestellt wird. Trotz Verdünnungsmittel ($N_2$) werden bei 400°C mehr unerwünschte $C_9$-$C_{11}$-Kohlenwasserstoffe gebildet als erwünschtes Propylen.

Ein ähnliches Verfahren ist aus der US-A-4 471 150 bekannt. Bei diesem Verfahren wird ein mit Magnesiumoxid, Manganoxid oder Magnesiumoxid/Platinoxid modifiziertes kristallines Alumosilikat, z.B. ein Zeolith vom Typ ZSM-34 in der H-Form verwendet. Die Umsetzung des Methanol- und/oder Dimethyletherdampfes kann in einem Festbett oder in einem Fließbett mit Verdünnungsmitteln, wie Wasserdampf, durchgeführt werden, wobei vorzugsweise mehr als 0,5 Mol Wasser je Mol organische Reaktanten eingesetzt werden. Der Gesamtdruck soll vorzugsweise zwischen etwa 0,37 und 3,0 bar liegen, wobei besonders bevorzugt bei Atmosphärendruck gearbeitet wird.

Der Propylengehalt des erzeugten Olefingemisches liegt lediglich zwischen 21 und 29 Gew.-%. Es wurde offenbar nicht erkannt, daß durch eine Verminderung des Gesamtdruckes der Propylenanteil erhöht werden kann; ferner hatte der verwendete Katalysator auch in der H-Form noch einen verhältnismäßig hohen Alkaligehalt (350 ppm Na, 1,47 % K).

Aus der US-A-4 025 575 ist ferner ein Verfahren bekannt, wonach Methanol und/oder Dimethylether mit einem Verdünnungsmittel, wie Stickstoff, Methan oder Wasserdampf, über Katalysatoren auf der Basis von kristallinen Alumosilikaten, insbesondere über Zeolithkatalysatoren vom HZSM5-Typ geleitet werden. Die Verwendung von Verdünnungsmitteln bedingt eine Herabsetzung des Methanol/Dimethylether-Partialdruckes auf weniger als Atmosphärendruck. Obwohl erwähnt ist, daß der Gesamtdruck in der Reaktionszone unterhalb Atmosphärendruck liegen kann, liegt er vorzugsweise zwischen etwa 1 und 35 bar. Mit Wasser als Verdünnungsmittel bei einem Gesamtdruck von 1 bar und einem Methanol-Partialdruck von 0,50 bar (Beispiel 19) betrug der Propylengehalt im Endprodukt nur 33,75 Gew.-% und der Anteil der Olefine mit 5 oder weniger Kohlenstoffatomen ($C_5$-Olefine) nur 64,10 Gew.-%. Bei Verwendung von Stickstoff (Beispiel 7) war der Propylen-Anteil mit 47,98 Gew.-% sowie der Anteil der $C_5$-Olefine mit 86,77 Gew.-% am höchsten. Die Verwendung von Stickstoff als Verdünnungsmittel hat jedoch den Nachteil, daß die Auftrennung des Reaktionsproduktes schwierig ist, da die niederen Olefine nur bei sehr niedrigen Temperaturen auskondensiert werden können.

Eine Abtrennung der niederen Olefine vom Verdünnungsmittel ist natürlich einfacher, wenn Wasser als Verdünnungsmittel verwendet wird, da dieses leicht aus dem Reaktionsgemisch auskondensiert werden kann.

Der Erfindung liegt deshalb die Aufgabe zugrunde, bei einem Verfahren der eingangs definierten Gattung die Ausbeute an niederen Olefinen, insbesondere von Propylen, zu verbessern und gleichzeitig die Abtrennung der niederen Olefine vom Verdünnungsmittel zu erleichtern.

Diese Aufgabe wird erfindungsgemäß dadurch gelöst, daß man zur Erzeugung eines Olefingemisches mit mindestens 5 Gew.-% Ethylen, mindestens 35 Gew.-% Propylen und höchstens 30 Gew.-% Butylen, bezogen auf die Gesamtkohlenwasserstoffe, die Umsetzung

(a) bei einem Gesamtdruck von 10 bis 90 kPa,

(b) bei einem Gewichtsverhältnis zwischen Wasser und Methanol bzw. Methanoläquivalenten von 0,1 bis 1,5

(c) bei einer Temperatur des Reaktor-Kühlmediums von 280 bis 570°C

(d) an einem Protonen enthaltenden Katalysator vom Pentasil-Typ mit einem Alkaligehalt von weniger als 380 ppm (vorzugsweise von weniger als 200 ppm, insbesondere von weniger als 100 ppm), einem ZnO-Gehalt von weniger als 0,1 Gew.-%, einem CdO-Gehalt von weniger als 0,1 Gew.-%, einer BET-Oberfläche von 300 bis 600 m²/g und einem Porenvolumen (nach der Quecksilberporosimetrie bestimmt) von 0,3 bis 0,8 cm³/g durchführt.

Ein "Methanoläquivalent" entspricht erfindungsgemäß nach der Gleichung

$$2\ CH_3OH \rightarrow CH_3\text{-}O\text{-}CH_3 + H_2O$$

einen halben Mol Dimethylether. Das nach der vorstehenden Gleichung gebildete Wasser wird dem unter (b) angegebenen Verhältnis nicht zugerechnet. Das Verhältnis zwischen Methanoldampf und Dimethyletherdampf kann in weiten Grenzen variiert werden, d.h. es können im Extremfall 100 % Methanol bzw. 100 % Dimethylether eingesetzt werden. Bei Verwendung eines hohen Anteils an Dimethylether, der in einer Vorstufe an einem sauren Katalysator aus Methanol erzeugt werden kann, ist die Reaktionswärme der Olefinerzeugungsreaktion in der Hauptstufe niedriger als bei Verwendung eines hohen Methanolanteils. Durch Vorschalten der ersten Stufe und gegebenenfalls einer zusätzlichen Kühlstufe ist es also möglich, die stark exotherme Olefinbildungsreaktion in der Hauptstufe zu steuern, so daß der Temperaturanstieg im Katalysator der Hauptstufe reduziert werden kann. Es kann aber auch reines Methanol eingesetzt werden, insbesondere wenn die Temperatur des Reaktor-Kühlmediums an der unteren Grenze (Merkmal c) liegt bzw. wenn der Gesamtdruck (Merkmal a) niedrig oder das Gewichtsverhältnis zwischen Wasser und Methanol (Merkmal b) hoch ist.

Das erfindungsgemäße Verfahren ergibt durch eine Kombination aller Merkmale eine überraschend hohe Propylenausbeute. Überraschend war auch, daß eine Absenkung des Methanol-Partialdrucks allein nicht ausreicht, sondern auch die Absenkung des Gesamtdruckes erforderlich ist. Unter diesen Umständen bleibt die Umsetzung offenbar bei den niederen Olefinen stehen, d.h. es findet keine Reaktion zu höheren Olefinen statt. Wesentlich ist offenbar auch, daß der Katalysator genügend saure Zentren besitzt, d.h. daß der Alkaligehalt sehr niedrig ist.

Besonders vorteilhafte Ergebnisse werden erzielt, wenn man die Umsetzung bei einem Gesamtdruck von 20 bis 70, vorzugsweise von 30 bis 60 kPa, durchführt.

Vorzugsweise führt man die Umsetzung bei einem Gewichtsverhältnis Wasser/Methanol bzw. Methanoläquivalenten von 0,2 bis 1,0, insbesondere von 0,3 bis 0,8, durch.

Vorzugsweise führt man die Umsetzung bei einer Temperatur des Reaktor-Kühlmediums vom 300 bis 570, insbesondere von 350 bis 500°C, durch.

Nach einer besonders bevorzugten Ausführungsform wird die Umsetzung in einem Salzbad-Röhrenreaktor durchgeführt, wobei die Temperatur im Katalysator die Temperatur des Reaktor-Kühlmediums um nicht mehr als 60°C überschreiten soll. Infolge der exothermen Reaktion bildet sich entlang der Katalysatorfüllung ein Temperaturpeak aus, der möglichst niedrig gehalten werden sollte, da bei einer Erhöhung der Temperatur im Katalysator die Neigung des zunächst gebildeten Ethylens zur Weiterreaktion zunimmt.

Eine wichtige Rolle bei der Erhöhung der Propylenausbeute spielt auch die Struktur des verwendeten Katalysators. Diese ist vorzugsweise aus Primärkristalliten mit einem mittleren Durchmesser von mindestens 0,1 μm und höchstens 0,9 μm aufgebaut, die teilweise zu Agglomeraten vereinigt sind, wobei die Primärkristallite bzw. Agglomerate durch feinteiliges Aluminiumoxid miteinander verbunden sind. Das Aluminiumoxid ist vorzugsweise durch Hydrolyse von aluminiumorganischen Verbindungen erhältlich. Die Agglomerate des Katalysators haben aufgrund dieser Struktur ein hohes Porenvolumen und verhältnismäßig große Poren, wodurch die zunächst gebildeten niederen Olefine leicht abdiffundieren können, so daß Weiterreaktionen an der Katalysatoroberfläche zurückgedrängt werden. Die verwendeten Katalysatoren haben also bei einer hohen Aktivität auch eine hohe Selektivität.

Vorzugsweise setzt man einen Katalysator ein, bei dem der mittlere Durchmesser der Primärkristallite im Bereich von 0,1 bis 0,6 μm liegt. Wenn der mittlere Durchmesser kleiner als 0,1 μm ist, vermindert sich die Lebensdauer der Katalysatoren beträchtlich, während bei einem Durchmesser von mehr als 0,9 μm die An-

3

fangsaktivität sehr niedrig ist. Der mittlere Durchmesser der Primärkristallite wird definiert als das über eine Vielzahl von Kristalliten gemittelte arithmetische Mittel zwischen dem größten und dem kleinsten Durchmesser eines einzelnen Kristalliten. Diese Definition hat ihre Bedeutung bei Kristalliten mit einem unregelmäßigen Kristallhabitus, z.B. bei stäbchenförmigen Kristalliten. Bei kugelförmigen bzw. annähernd kugelförmigen Kristalliten fallen der größte und der kleinste Durchmesser zusammen.

Die Primärkristallite können in den erfindungsgemäß verwendeten Katalysatoren zu mindestens 20 % zu Agglomeraten von 5 bis 500 µm vereinigt sein. Bei den angegebenen Werten handelt es sich wieder um die mittleren Abmessungen (arithmetisches Mittel aus der größten und der kleinsten Abmessung, gemittelt über eine Vielzahl von Kristalliten).

Die Verbundkörper haben im allgemeinen Abmessungen von 20 bis 1000 µm, insbesondere von 50 bis 800 µm. Auch bei diesen Werten handelt es sich um mittlere Abmessungen, die wie vorstehend angegeben, definiert sind.

Der Aufbau des Katalysators aus Primärkristalliten, Agglomeraten und Bindemittelteilchen bestimmt auch die BET-Oberfläche (300 bis 600 $m^2/g$), das Porenvolumen (0,3 bis 0,8 $cm^3/g$) sowie den Porendurchmesser, d.h. mindestens 20 % der Poren haben vorzugsweise einen Durchmesser von 14 bis 80 nm.

Die BET-Oberfläche, das Porenvolumen und der Porendurchmesser stellen eine optimale Auswahl dar, um Katalysatoren mit hoher Aktivität, Selektivität und Lebensdauer zu erhalten.

Die Menge des feinteiligen Aluminiumoxid-Bindemittels beträgt vorzugsweise 10 bis 40 Gew.-%, bezogen auf das Gewicht des Endproduktes.

Vorzugsweise liegt das feinteilige Aluminiumoxid-Bindemittel im Reaktionsansatz als peptisierbares Aluminiumoxidhydrat vor, wovon mindestens 95 % der Teilchen (auf den mittleren Durchmesser bezogen) $\leq$ 55 µm sind. Das feinteilige Aluminiumoxid-Bindemittel ist vorzugsweise durch Hydrolyse von Aluminiumtrialkylen oder Aluminiumalkoholaten erhältlich.

Der erfindungsgemäß verwendete Katalysator ist vorzugsweise auf folgende Weise erhältlich:

(a) in einem wäßrigen Reaktionsansatz, enthaltend eine Siliciumquelle, eine Aluminiumquelle, eine Alkaliquelle und ein Templat, wird bei erhöhter Temperatur und gegebenenfalls bei erhöhtem Druck in an sich bekannter Weise ein alkalisches Alumosilicatgel erzeugt und in ein kristallines Alumosilicat umgewandelt, wobei aber die Reaktion abgebrochen wird, wenn die erhaltenen Primärkristallite einen mittleren Durchmesser von mindestens 0,1 µm und höchstens 0,9 µm, vorzugsweise von 0,1 bis 0,6 µm haben;

(b) die Primärkristallite werden aus dem wäßrigen Reaktionsmedium als Voragglomerate abgetrennt, getrocknet und einer zwischencalcinierung unterzogen;

(c) das Produkt von Stufe (b) wird zum Austausch der Alkaliionen in wäßrigem Medium mit einer protonenhaltigen oder beim Erhitzen Protonen liefernden Substanz umgesetzt bis der Alkaligehalt unter 380 ppm, vorzugsweise unter 200 ppm, insbesondere unter 100 ppm, abgesunken ist, abgetrennt, getrocknet, und erneut einer zwischencalcinierung unterzogen, worauf eine Agglomeratfraktion von etwa 5 bis 500 µm abgetrennt wird;

(d) die Agglomeratfraktion von Stufe (c) wird mit dem feinteiligen Aluminiumoxidhydrat vermischt;

(e) das Produkt von Stufe (d) wird einer Abschlußcalcinierung unterzogen.

Die Bedeutung der einzelnen Stufen, nach denen der erfindungsgemäß verwendete Katalysator erhältlich ist, ist nachstehend näher erläutert:

In der Stufe (a) wird zunächst ein wäßriger Reaktionsansatz, enthaltend eine Siliciumquelle (beispielsweise kolloidale Kieselsäure oder ein Alkalisilikat), eine Aluminiumquelle (beispielsweise Aluminiumhydroxid oder Natriumaluminat), eine Alkaliquelle (beispielsweise ein Alkalihydroxid, wobei die Alkaliquelle bei Verwendung von Alkalisilicaten auch ein Teil der Siliciumquelle und bei Verwendung von Alkalialuminaten auch ein Teil der Aluminiumquelle sein kann) und ein Templat, hergestellt. Die Gewichtsanteile zwischen Siliciumquelle und Aluminiumquelle werden so gewählt, daß kristalline Alumosilikate mit einem Si/Al-Atomverhältnis von mindestens 10, vorzugsweise von etwa 20 bis 500 : 1 erhalten werden. Aus dem Reaktionsansatz wird bei erhöhter Temperatur und gegebenenfalls bei erhöhtem Druck in an sich bekannter Weise ein alkalisches Alumosilikatgel erzeugt. Man kann schon bei Temperaturen ab 90°C arbeiten, doch werden in diesem Fall die Reaktionszeiten verhältnismäßig lang (etwa 1 Woche). Es wird deshalb vorzugsweise bei Temperaturen von 90 bis 190°C, insbesondere von 90 bis 150°C gearbeitet, wobei sich bei Temperaturen von mehr als 100°C (unter Normalbedingungen) in Abhängigkeit von der Temperatur automatisch ein Überdruck einstellt.

Das Alumosilikatgel wandelt sich im Laufe der Reaktion in ein kristallines Alumosilikat um. Wenn die Temperatur des Reaktionsansatzes höher als 190°C liegt, wird das Wachstum der Alumosilikat-Primärkristallite zu schnell, und es werden Primärkristallite mit einem Durchmesser von mehr als 0,9 µm erhalten, während gleichzeitig noch Alumosilicatgel im Reaktionsansatz vorhanden ist.

Als Template werden Tetraalkylammoniumverbindungen, vorzugsweise Tetrapropylammoniumhydroxid (TPAOH) oder Tetrapropylammoniumbromid (TPABr) eingesetzt. Man kann als Template auch Gemische aus Ammoniak oder einem organischen Amin und einer weiteren organischen Verbindung aus der Gruppe der Alkohole, vorzugsweise Butanol, verwenden.

Der wäßrige Reaktionsansatz von Stufe (a) hat vorzugsweise einen pH-Wert von 10 bis 13. Bei einem pH-Wert von weniger als 10 verläuft die Umwandlung des Alumosilikatgels in das kristalline Alumosilikat verhältnismäßig langsam. Bei höheren pH-Werten als 13 können sich die Alumosilikatkristalle in einigen Fällen wieder auflösen. Dies kann aber im allgemeinen toleriert werden, weil sich in der Regel zunächst nur die kleineren Primärkristallite mit einem Durchmesser von weniger als 0,1 µm wieder auflösen.

Die Bildung der kristallinen Alumosilikat-Primärkristallite kann durch geeignete Auswahl der Siliciumquelle, der Aluminiumquelle, der Alkaliquelle und des Templats sowie durch geeignete Auswahl der Temperatur und des pH-Werts und der Rührgeschwindigkeit geregelt werden. Wesentlich ist, daß die Reaktion abgebrochen wird, wenn die erhaltenen Primärkristallite einen mittleren Durchmesser von mindestens 0,1 µm und höchstens 0,9 µm haben.

Zu diesem Zweck werden mehrere Testansätze durchgeführt. Schon nach wenigen Versuchen lassen sich die optimalen Parameter ermitteln, aufgrund derer die erforderlichen Größenbereiche der Primärkristallite erreicht werden. Ein Indiz für die Beendigung der Reaktion besteht auch darin, daß der pH-Wert des Reaktionsansatzes sprunghaft ansteigt.

Erfindungsgemäß braucht nicht in jedem Fall ein neuer Reaktionsansatz hergestellt zu werden. Vielmehr kann zur Erzeugung des Alumosilicatgels die Siliciumquelle, die Alkaliquelle, die Aluminiumquelle, das Templat und das Wasser aus den Mutterlaugen vorheriger Synthesen verwendet und durch die für die Synthese des Alumosilicatgels erforderlichen Mengen der genannten Verbindungen ergänzt werden.

Die Bildung der Alumosilicat-Primärkristallite von Stufe (a) erfolgt vorzugsweise bei einem pH-Wert zwischen 10 und 13, wobei der Reaktionsansatz gerührt wird. Auf diese Weise wird die Größenverteilung der Primärkristallite homogenisiert. Die Rührgeschwindigkeit soll aber vorzugsweise nicht mehr als 900 U/min betragen. Bei größeren Rührgeschwindigkeiten ist der Anteil der kleineren Primärkristallite höher, so daß die Reaktionszeit verlängert werden muß, damit gewährleistet ist, daß der mittlere Durchmesser aller Primärkristallite mindestens 0,1 µm beträgt.

In der Stufe (b) werden die Primärkristallite aus dem wäßrigen Reaktionsmedium als Voragglomerate abgetrennt, d.h. nicht als Einzelkristallite. Dies wird vorzugsweise dadurch erreicht, daß man dem wäßrigen Reaktionsmedium ein Flockungsmittel zusetzt. Im allgemeinen wird als Flockungsmittel eine kationische organische makromolekulare Verbindung, vorzugsweise ein Copolymer aus Acrylamid und einem kationischen Acrylsäurederivat, verwendet.

Das Flockungsmittel erleichtert nicht nur die Abtrennung der Primärkristallite aus dem Reaktionsmedium (verbesserte Filtrierbarkeit), sondern bewirkt auch, daß sich die Primärkristallite zu Voragglomeraten zusammenschließen, die hinsichtlich Größe, Struktur und Anlagerung der Primärkristallite schon weitgehend den in der folgenden Stufe gebildeten Agglomeraten gleichen. Die Voragglomerate werden getrocknet und einer Zwischencalcinierung unterzogen, die zunächst vorzugsweise in einer inerten Atmosphäre bei etwa 200 bis 350°C, insbesondere bei etwa 250°C durchgeführt wird, wobei ein Teil des Templats desorbiert wird.

Die Zwischencalcinierung kann dann in einer oxidierenden Atmosphäre bei etwa 500 bis 600°C vervollständigt werden, wobei die gegebenenfalls noch vorhandene Restmenge an Templat abgebrannt wird.

Im allgemeinen werden die Voragglomerate etwa 1 bis 20 Stunden in der inerten Atmosphäre und etwa 1 bis 30 Stunden in der oxidierenden Atmosphäre zwischencalciniert.

In der Stufe (c) wird das Produkt von Stufe (b) zum Austausch der Alkaliionen in wäßrigem Medium mit einer protonenhaltigen oder beim Erhitzen Protonen liefernden Substanz umgesetzt. Beispielsweise kann der Ionenaustausch mit Hilfe einer verdünnten Mineralsäure (z.B. Salzsäure oder Schwefelsäure) oder einer organischen Säure (z.B. Essigsäure) durchgeführt werden. Der Ionenaustausch erfolgt vorzugsweise unter Rühren mindestens eine Stunde bei Temperaturen zwischen 25 und 100°C, wobei zumindest ein Teil der Alkaliionen in den Voragglomeraten der Primärkristallite durch Wasserstoffionen ausgetauscht werden. Falls erforderlich, kann der Ionenaustausch unter den gleichen Bedingungen wiederholt werden.

Nach dem Austausch der Alkaliionen im wäßrigen Medium wird das Protonen enthaltende Produkt (H-Zeolith) abgetrennt (beispielsweise durch Filtration), getrocknet und erneut einer Zwischencalcinierung unterzogen. Die Zwischencalcinierung wird bei Temperaturen von 400 bis 800°C, vorzugsweise bei etwa 600°C über einen Zeitraum von 5 bis 20 Stunden durchgeführt.

Statt mit der verdünnten Säure kann der Ionenaustausch auch mit Hilfe einer Ammoniumsalzlösung unter vergleichbaren Bedingungen durchgeführt werden. In diesem Fall werden die Alkaliionen durch Ammoniumionen ausgetauscht. Wird das so erhaltene Produkt zwischencalciniert, so wird Ammoniak entfernt, und man erhält ein Protonen enthaltendes Produkt.

Das nach der Zwischencalcinierung erhaltene Produkt enthält einerseits Agglomerate, die $\geqq 500$ µm sind, und andererseits Staubanteile, die $\leqq 5$ µm sind. Es wird daher eine Agglomeratfraktion von etwa 5 bis 500

μm abgetrennt.

Diese Agglomeratfraktion wird in der Stufe (d) mit dem feinteiligen Aluminiumoxidhydrat vermischt, von dem mindestens 95 % ≦ 55 μm und mindestens 30 % ≧ 35 μm sind. Diese Werte sind, gemittelt über eine Vielzahl von Kristalliten, jeweils auf den mittleren Durchmesser bezogen, der wie der mittlere Durchmesser der Primärkristallite definiert ist. Im einzelnen hat das Aluminiumoxid typischerweise folgendes Kornspektrum:

99 % ≦ 90 μm

95 % ≦ 45 μm

55 % ≦ 25 μm.

Das Aluminiumoxidhydrat ist im wesentlichen für die Einstellung des Porenvolumens des erfindungsgemäßen Katalysators verantwortlich. Die Menge des feinteiligen Aluminiumoxidhydrat-Bindemittel beträgt vorzugsweise etwa 10 bis 70 Gew.-%, bezogen auf das Gesamtgewicht des Produktes von Stufe (d). Vorzugsweise ist das feinteilige Aluminiumoxidhydrat-Bindemittel peptisierbares Aluminiumoxid, das besonders Na- und Fe-arm ist.

Die Peptisierung erfolgt vorzugsweise mit einer verdünnten schwachen Säure.

Das Produkt von Stufe (d) wird einer Abschlußcalcinierung unterzogen. Diese kann bei Temperaturen von etwa 400 bis 800°C, vorzugsweise bei etwa 540°C, über einen Zeitraum von 5 bis 20 Stunden durchgeführt werden.

Der erfindungsgemäß bevorzugt verwendete Katalysator ist in der EP-A-369364 im einzelnen beschrieben.

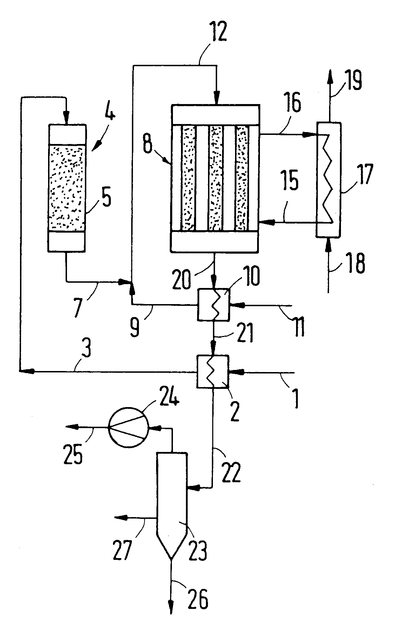

Das erfindungsgemäße Verfahren ist anhand der Zeichnung erläutert.

Methanol wird in der Leitung 1 herangeführt, im Wärmeaustauscher 2 auf Temperaturen von etwa 250 bis 350°C erhitzt und dadurch verdampft. In der Leitung 3 führt man den Methanoldampf zu einem Vorreaktor 4, der eine Schüttung 5 aus einem körnigen Dehydratisierungskatalysator enthält. Als Katalysator kann z.B. Aluminiumoxid verwendet werden, wie es in der US-PS 4 058 576 beschrieben ist. Im Vorreaktor 4 wird ein Teil des Methanols zu Dimethylether und Wasser umgewandelt, das entstehende Gemisch zieht man in der Leitung 7 ab.

Durch die Umsetzung im Vorreaktor 4 wird die Wärmeerzeugung im nachfolgenden Röhrenreaktor 8 abgemildert. Dem Gemisch der Leitung 7 gibt man durch die Leitung 9 eine dosierte Menge an Wasserdampf zu, der vom Wärmeaustauscher kommt. Kühlwasser zum Erzeugen des Wasserdampfs wird dem Wärmeaustauscher 10 in der Leitung 11 zugeführt. Dem Röhrenreaktor 8 gibt man durch die Leitung 12 ein Gemisch aus Methanol, Dimethylether und Wasserdampf auf, dessen Temperatur im Bereich von etwa 250 bis 450°C liegt. Das Gemisch der Leitung 12 weist ein Wasser/Methanoläquivalent-Gewichtsverhältnis von 0,1 bis 1,5, vorzugsweise 0,2 bis 1,0 und besonders vorzugsweise von höchstens 0,8 auf, dabei ist 1 Mol DME = 2 Mol Methanoläquivalent. Im Röhrenreaktor 8 ist der Katalysator in zahlreichen Röhren angeordnet, üblicherweise haben die Röhren eine Länge von 1 bis 5 m und einen inneren Durchmesser von 20 bis 50 mm.

Für die Umsetzung von Methanol und DME zu den gewünschten niederen Olefinen, insbesondere Ethylen und Propylen, ist einerseits eine gute Kühlung des Katalysators und andererseits ein unteratmosphärischer Gesamtdruck im Katalysatorbereich von 10 bis 90 kPa, vorzugsweise 20 bis 70 kPa und besonders vorzugsweise von 30 bis 60 kPa zu empfehlen. Die indirekte Kühlung des Katalysators, der in den Röhren angeordnet ist, erfolgt durch ein Salzbad. Das Salzbad wird in der Leitung 15 zum Reaktor 8 geführt, es verläßt den Reaktor in der Leitung 16, gibt Wärme in einem Wärmeaustauscher 17 ab und strömt zurück zum Reaktor 8. Der Wärmeaustauscher 17 erhält durch die Leitung 18 Speisewasser, in der Leitung 19 zieht man Hochdruckdampf ab.

Die Umsetzung am Katalysator des Röhrenreaktors 8 erfolgt bei Temperaturen im Bereich von 280 bis 570°C, vorzugsweise bei mindestens 300°C und besonders vorzugsweise im Temperaturbereich von 350 bis 500°C. Bei Verwenden einer Salzschmelze als Kühlmedium ist es möglich, die Temperatur am Katalysator bei nicht mehr als 60°C über der Temperatur des Salzbades zu halten. Dadurch werden Schädigungen des Katalysators durch zu hohe Spitzentemperaturen vermieden, überraschend hat sich auch gezeigt, daß dadurch die Ausbeute an Propylen erhöht und die Erzeugung von Butylen in erwünschter Weise verringert wird.

Das Produktgemisch verläßt den Röhrenreaktor 8 in der Leitung 20, erfährt eine erste Kühlung im Wärmeaustauscher 10, strömt dann durch die Leitung 21 zum Wärmeaustauscher 2, wobei das Produkt teilweise kondensiert. Durch die Leitung 22 führt man das Produkt zu einem Trennbehälter 23, aus welchem die dampfförmigen, niederen Olefine durch die Vakuumpumpe 24 abgesaugt werden und als Produktgemisch in der Leitung 25 zur Verfügung stehen. Das sich im Behälter sammelnde Kondensat trennt sich in Wasser und Benzinkohlenwasserstoffe, wobei man das Wasser in der Leitung 26 und die Benzinkohlenwasserstoffe in der Leitung 27 abzieht.

Die Erfindung ist durch die nachstehenden Beispiele erläutert.

Herstellungsbeispiel

Nach diesem Beispiel wurden Alumosilikat-Zeolithe mit einer Primärkristallitgröße von < 1 μm hergestellt. Im einzelnen wurde wie folgt verfahren:

Eine Reaktionsmischung wurde durch inniges Mischen zweier Lösungen bei Raumtemperatur in einem 1000 Liter-Druckrührbehälter hergestellt. Die beiden Lösungen wurden als Lösung A und Lösung B bezeichnet. Die Lösung A wurde hergestellt, indem in 600 Liter deionisiertem Wasser 95,7 kg TPABr gelöst wurden. In diese Lösung wurden 215,8 kg einer handelsüblichen Kieselsäure eingetragen. Die Lösung B wurde hergestellt, indem in 50 Liter deionisiertem Wasser 33,1 kg NaOH und anschließend 2 kg NaAlO$_2$ gelöst wurden. Die noch warme Lösung B wurde zur Lösung A gegeben. Es wurden noch 62 Liter deionisiertes Wasser zugefügt. Der Autoklav wurde dann geschlossen und unter Rühren bei etwa 600 bis 800 U/min. auf die Reaktionstemperatur gebracht. Nach etwa 50 Stunden war die Reaktion beendet, wie aus dem pH-Sprung (von 11,6 auf 12,2) zu ersehen war. Nach dem Abkühlen wurde der Druckbehälter geöffnet, das Produkt dem Reaktionskessel entnommen und filtriert. Der Filterkuchen wurde aufgeschlämmt, mit einer 0,4 Gew.-%igen wäßrigen Suspension eines handelsüblichen Flockungsmittels versetzt und nach dem Rühren und Absetzen der Voragglomerate des Feststoffes dekantiert. Der beschriebene Waschprozeß wurde wiederholt, bis das Waschwasser einen pH-Wert von 7 bis 8 und eine Br$^-$-Konzentration von weniger als 1 ppm hatte. Die Aufschlämmung, in der Voragglomerate von Primärkristalliten zu erkennen waren, die offenbar durch das Flockungsmittel zusammengehalten wurden, wurde filtriert. Der gut filtrierbare Filterkuchen wurde anschließend bei 120°C 12 Stunden getrocknet und bei 540°C 24 Stunden calciniert. Die Größe der Primärkristallite ist in Tabelle I angegeben. In Reihenversuchen waren vorher die zur Erzielung der angestrebten Kristallitgrößen erforderlichen Bedingungen ermittelt worden.

Der getrocknete Filterkuchen wurde vor der Calcinierung mit einem handelsüblichen Granulator auf eine Korngröße von kleiner als 2 mm zerkleinert.

Das Granulat wurde mit einer Aufheizrate von 1°C/min. unter Stickstoff (1000 Nl/h) auf 350°C gebracht und bei 350°C 15 Stunden unter Stickstoff (1000 Nl/h) calciniert. Dann wurde die Temperatur mit einer Aufheizrate von 1°C/min auf 540°C erhöht, und das Granulat wurde 24 Stunden bei dieser Temperatur an Luft calciniert, um das restliche TPA abzubrennen. Der calcinierte Na-Zeolith wurde analysiert, wobei die in Tabelle I angegebenen Ergebnisse erhalten wurden.

Der calcinierte Na-Zeolith wurde in der 5-fachen Menge einer 1-molaren wäßrigen HCl-Lösung suspendiert und auf 80°C gebracht. Bei dieser Temperatur wurde eine Stunde gerührt. Dann wurde eine 0,4 Gew.-%ige Suspension des Flockungsmittels hinzugegeben, und die überstehende Säure wurde nach Absitzen des Feststoffes abdekantiert. Der so beschriebene Vorgang wurde noch einmal wiederholt.

Der Feststoff wurde in etwa 10 Waschvorgängen jeweils in etwa 300 Liter deionisiertem Wasser unter Rühren suspendiert und mit einer 0,4 Gew.-%igen Suspension des Flockungsmittels versetzt. Nach dem Absitzen des Zeolithen wurde die überstehende Lösung dekantiert. Als der Gehalt an Cl$^-$ im Waschwasser < 5 ppm war, wurde die Suspension abfiltriert und bei 120°C 15 Stunden getrocknet. Der Natriumgehalt betrug etwa 340 ppm, der Kaliumgehalt lag unter 20 ppm.

Der getrocknete H-Zeolith wurde mit einem handelsüblichen Granulator auf < 2 mm zerkleinert und unter Luft mit einer Aufheizrate von 1°C/min auf 540°C gebracht und bei dieser Temperatur unter Luft 10 Stunden calciniert. Die Spezifikation dieses calcinierten H-Zeolithen ist in Tabelle I angegeben.

5000 g des calcinierten H-Zeolithen wurden mit Hilfe einer Labormühle auf eine Korngröße von <500 μm gemahlen und in einem Doppel-Z-Kneter mit 1470 g eines handelsüblichen peptisierbaren Aluminiumoxidhydrats mit einem Korngrößenspektrum von

99 Gew.-% $\leqq$ 90 μm
95 Gew.-% $\leqq$ 45 μm
55 Gew.-% $\leqq$ 25 μm

15 Min. trocken gemischt. Zu dieser Mischung wurde langsam eine 17 Gew.-%ige wäßrige Salpetersäurelösung (zur Peptisierung des Aluminiumoxidhydrats) und Steatitöl gegeben.

Diese Mischung wurde etwa 30 min. bis zur Plastifizierung geknetet und in einem handelsüblichen Extruder zu Formkörpern mit einem Durchmesser von etwa 3 mm und einer Länge von 6 mm extrudiert. Die Abschlußcalcinierung wurde 5 Stunden bei 600°C durchgeführt.

Die Analysenwerte sowie die physikalischen und chemischen Eigenschaften des Produkts sind in Tabelle I angegeben.

Tabelle I

| Molverhältnis der Ausgangsstoffe | |
|---|---|
| $SiO_2$ | 100 |
| $NaAlO_2$ | 0,67 |
| NaOH | 23 |
| TPABr | 10 |
| $H_2O$ | 1100 |

| Kristallisationsdaten | |
|---|---|
| Temp.(°C) | 130 |
| Zeit (h) | 50 |
| Kristallinität (%) | 100 |
| Primärkristallitgröße (µm) | 0,3 |

| Si- und Al-Gehalt des Na-Zeolithen | |
|---|---|
| Si (Gew.-%) | 45,0 |
| Al (Gew.-%) | 0,42 |
| Si/Al (Atomverhältnis) | 103 |

| Phys. und chem. Eigenschaften des Katalysators | |
|---|---|
| BET-Oberfläche ($m^2/g$) | 342 |
| Porenvolumen ($cm^3/g$) | 0,33 |
| Poren > 80 nm (%) | 25,1 |
| Poren 14 - 80 nm (%) | 68,1 |
| Na-Gehalt (ppm) | ca. 340 |

Anwendungsbeispiel 1

Einem aus dem Vorreaktor 4 erhaltenen Gemisch aus Methanol- und Dimethyletherdampf wurde über die Leitung 9 der Wasserdampf zugesetzt. Die Temperatur des Gemisches betrug vor Eintritt in den Röhrenreaktor 8 400°C. Das Gewichtsverhältnis zwischen zugesetztem Wasserdampf und Methanoldampf (Leitung 3) betrug 1,0; die Raumgeschwindigkeit betrug 0,5 kg insgesamt eingesetztes Methanol je kg Katalysator und Stunde. Das aus dem Röhrenreaktor 8 austretende Produktgemisch wurde zunächst im Wärmeaustauscher 10 gekühlt und anschließend im Wärmeaustauscher 2 teilweise auskondensiert. Die niederen Olefine wurden über die Leitung 25 abgeführt. Das im Behälter 23 aufgefangene Kondensat trennt sich in Wasser und Benzinkohlenwasserstoffe, wobei das Wasser durch die Leitung 26 und die Benzinkohlenwasserstoffe durch die Leitung 27 abgezogen werden.

Der Röhrenreaktor 8 wurde in Form eines Einzelrohrs betrieben, das mit 1,2 Liter des Katalysators gemäß Herstellungsbeispiel gefüllt war. Die Temperatur im Salzbad betrug 439°C, das Salz bestand aus einer Mischung aus 44 Mol-% $KNO_3$, 7 Mol-% $NaNO_2$ und 49 Mol-% $NaNO_3$.

Der Katalysator im Salzbadreaktor wurde auf einem unteratmosphärischen Gesamtdruck von 50 kPa gehalten, indem die gebildeten Reaktionsprodukte fortlaufend über die Vakuumpumpe 24 abgesaugt wurden.

Die Ergebnisse sind in Tabelle II angegeben.

## Anwendungsbeispiel 2

Die Arbeitweise von Anwendungsbeispiel 1 wurde mit der Abweichung wiederholt, daß die Temperatur des Salzbades auf 439°C eingestellt wurde, die Raumgeschwindigkeit 1,0 kg Methanol je kg Katalysator und Stunde und das Gewichtsverhältnis des in der Leitung 9 herangeführten Wasserdampfs zum Methanoldampf der Leitung 3 0,5 betrug. Die Ergebnisse sind ebenfalls in Tabelle II angegeben.

## Anwendungsbeispiel 3

Die Arbeitsweise von Anwendungsbeispiel 2 wurde mit der Abweichung wiederholt, daß der Gesamtdruck auf 40 kPa abgesenkt wurde.

Die Ergebnisse sind ebenfalls in Tabelle II angegeben.

### Tabelle II

| Anwendungsbeispiel | 1 | 2 | 3 |
|---|---|---|---|
| Druck (kPa) | 50 | 50 | 40 |
| Salzbadtemperatur (°C) | 428 | 439 | 439 |
| Maximaltemperatur im Kat (°C) | 458 | 475 | 468 |
| Raumgeschwindigkeit(kg/kg x h) | 0,5 | 1,0 | 1.0 |
| Wasserdampf/ | | | |
| Methanoldampf (kg/kg) | 1,0 | 0,5 | 0,5 |
| Methanol-Umwandlung (%) | 99,9 | 99,7 | 99,3 |

#### Kohlenwasserstoff-Verteilung im Produkt

| | | 1 | 2 | 3 |
|---|---|---|---|---|
| Paraffine ($C_1$-$C_4$) (Gew.-%) | | 5,58 | 5,10 | 4,75 |
| Olefine $C_2$ | (Gew.-%) | 7,27 | 6,82 | 6,00 |
| $C_3$ | (Gew.-%) | 42,14 | 44,37 | 47,63 |
| $C_4$ | (Gew.-%) | 25,66 | 23,95 | 22,49 |
| insgesamt | (Gew.-%) | 75,07 | 75,14 | 76,12 |
| Gasolin $C_5^+$ | (Gew.-%) | 19,35 | 19,76 | 19,13 |

## Anwendungsbeispiele 4 bis 8 (Vergleich)

Die Arbeitsweise von Anwendungsbeispiel 1 wurde wiederholt, wobei die Parameter jedoch, wie in Tabelle III angegeben, variiert wurden. Insbesondere betrug der Gesamtdruck der Reaktanten 100 kPa (1,0 bar), die Raumgeschwindigkeit lag stets bei 0,5 kg/kg x h und das Verhältnis des Wasserdampfs (Leitung 9) zum Methanoldampf (Leitung 3) betrug 1,0 kg/kg. Obwohl der Methanol-Partialdruck aufgrund des Wasserdampf-Methanol-Verhältnisses von 1,0 kg/kg nur 36 kPa betrug, ist dies nicht ausreichend, um die gewünschte hohe Propylenausbeute zu erzielen. Es ist vielmehr auch erforderlich, daß der Gesamtdruck der Reaktanten unterhalb Atmosphärendruck liegt.

Tabelle III

| Anwendungsbeispiel (Vergleich) | 4 | 5 | 6 | 7 | 8 |
|---|---|---|---|---|---|
| Salzbadtemperatur (°C) | 397 | 416 | 428 | 435 | 442 |
| Maximaltemperatur im Kat (°C) | 456 | 473 | 484 | 491 | 496 |
| **Methanol**-Umwandlung (%) | 99,9 | 99,9 | 99,9 | 99,9 | 99,9 |

Kohlenwasserstoff-Verteilung im Produkt

| | | 4 | 5 | 6 | 7 | 8 |
|---|---|---|---|---|---|---|
| Paraffine $C_1$-$C_4$ | (Gew.-%) | 13,02 | 11,51 | 10,68 | 10,08 | 9,92 |
| Olefine $C_2$ | (Gew.-%) | 6,46 | 8,09 | 8,84 | 10,21 | 10,46 |
| $C_3$ | (Gew.-%) | 20,87 | 25,77 | 28,23 | 29,96 | 31,21 |
| $C_4$ | (Gew.-%) | 18,48 | 20,08 | 20,77 | 20,62 | 21,17 |
| insgesamt | (Gew.-%) | 45,80 | 53,94 | 57,84 | 60,79 | 62,84 |
| Gasolin $C_5^+$ | (Gew.-%) | 41,18 | 34,55 | 31,48 | 29,13 | 27,24 |

## Patentansprüche

1. Verfahren zur Erzeugung von niederen Olefinen aus Methanol durch Umsetzung eines Methanol- und-/oder Dimethyletherdampf und Wasserdampf enthaltenen Reaktionsgemisches in einem Röhrenreaktor an einem indirekt gekühlten Katalysator auf der Basis von kristallinen Alumosilikaten vom Pentasil-Typ mit einem Si/Al-Atomverhältnis von mindestens 10,

   dadurch gekennzeichnet, daß man zur Erzeugung eines Olefingemisches mit mindestens 5 Gew.-% Ethylen, mindestens 35 Gew.-% Propylen und höchstens 30 Gew.-% Butylen, bezogen auf die Gesamtkohlenwasserstoffe, die Umsetzung

   (a) bei einem Gesamtdruck von 10 bis 90 kPa,

   (b) bei einem Gewichtsverhältnis zwischen Wasser und Methanol bzw. Methanoläquivalenten von 0,1 bis 1,5

   (c) bei einer Temperatur des Reaktor-Kühlmediums von 280 bis 570°C

   (d) an einem Protonen enthaltenden Katalysator vom Pentasil-Typ mit einem Alkaligehalt von weniger als 380 ppm, einem ZnO-Gehalt von weniger als 0,1 Gew.-%, einem CdO-Gehalt von weniger als 0,1 Gew.-%, einer BET-Oberfläche von 300 bis 600 m2/g und einem Porenvolumen (nach der Quecksilberporosimetrie bestimmt) von 0,3 bis 0,8 cm3/g durchführt.

2. Verfahren nach Anspruch 1, dadurch gekennzeichnet, daß man die Umsetzung bei einem Gesamtdruck von 20 bis 70, vorzugsweise von 30 bis 60 kPa, durchführt.

3. Verfahren nach Anspruch 1 oder 2, dadurch gekennzeichnet, daß man die Umsetzung bei einem Gewichtsverhältnis Wasser/Methanol bzw. Methanoläquivalenten von 0,2 bis 1,0, vorzugsweise von 0,3 bis 0,8, durchführt.

4. Verfahren nach einem der Ansprüche 1 bis 3, dadurch gekennzeichnet, daß man die Umsetzung bei einer Temperatur des Reaktor-Kühlmediums von 300 bis 570, vorzugsweise von 350 bis 500°C, durchführt.

5. Verfahren nach einem der Ansprüche 1 bis 4, dadurch gekennzeichnet, daß man die Umsetzung in einem Salzbad-Röhrenreaktor durchführt, wobei die Temperatur im Katalysator die Temperatur des Reaktor-Kühlmediums um nicht mehr als 60°C überschreitet.

6. Verfahren nach einem der Ansprüche 1 bis 5, dadurch gekennzeichnet, daß man die Umsetzung an einem Katalysator durchführt, der aus Primärkristalliten mit einem mittleren Durchmesser von mindestens 0,1 μm und höchstens 0,9 μm aufgebaut ist, die teilweise zu Agglomeraten vereinigt sind, wobei die Primärkristallite bzw. Agglomerate durch feinteiliges Aluminiumoxid miteinander verbunden sind.

7. Verfahren nach einem der Ansprüche 1 bis 6, dadurch gekennzeichnet, daß man einen Katalysator einsetzt, bei dem der mittlere Durchmesser der Primärkristallite im Bereich von 0,1 bis 0,6 μm liegt.

8. Verfahren nach einen der Ansprüche 1 bis 7, dadurch gekennzeichnet, daß man einen Katalysator einsetzt, dessen Primärkristallite zu mindestens 20 % zu Agglomeraten von 5 bis 500 μm vereinigt sind.

9. Verfahren nach einem der Ansprüche 1 bis 8, dadurch gekennzeichnet, daß man einen Katalysator einsetzt, von dem mindestens 20 % der Poren einen Durchmesser von 14 bis 80 nm besitzen.

## Claims

1. A process for the preparation of lower olefins from methanol by conversion of a reaction mixture containing methanol vapour and/or dimethyl ether vapour and water vapour in a tubular reactor on an indirectly cooled catalyst based on crystalline aluminosilicates of the pentasil type with a Si/Al atomic ratio of at least 10, characterised in that to prepare an olefin mixture with at least 5 % by weight of ethylene, at least 35 % by weight of propylene and at most 30 % by weight of butylene, in relation to the total hydrocarbons, the conversion is carried out
(a) at a total pressure of from 10 to 90 kPa,
(b) at a weight ratio between water and methanol and/or methanol equivalents of from 0.1 to 1.5,
(c) at a temperature of the reactor cooling medium of from 280 to 570°C,
(d) on a proton-containing catalyst of the pentasiltype with an alkali content of less than 380 ppm, a ZnO content of less than 0.1 % by weight, a CdO content of less than 0.1 % by weight, a BET surface area of from 300 to 600 $m^2/g$ and a pore volume (determined by mercury porosimetry) of from 0.3 to 0.8 $cm^3/g$.

2. A process according to Claim 1, characterised in that the conversion is carried out at a total pressure of from 20 to 70, preferably from 30 to 60 kPa.

3. A process according to Claim 1 or 2, characterised in that the conversion is carried out at a weight ratio of water/methanol or methanol equivalents of from 0.2 to 1.0, preferably from 0.3 to 0.8.

4. A process according to any one of Claims 1 to 3, characterised in that the conversion is carried out at a temperature of the reactor cooling medium of from 300 to 570, preferably from 350 to 500°C.

5. A process according to any one of Claims 1 to 4, characterised in that the conversion is carried out in a salt bath tubular reactor, wherein the temperature in the catalyst does not exceed the temperature of the reactor cooling medium by more than 60°C.

6. A process according to any one of Claims 1 to 5, characterised in that the conversion is carried out on a catalyst which is composed of primary crystallites with an average diameter of at least 0.1 μm and at most 0.9 μm, which are partly combined to form agglomerates, wherein the primary crystallites and/or agglomerates are bonded together by finely divided aluminium oxide.

7. A process according to any one of Claims 1 to 6, characterised in that a catalyst is used in which the average diameter of the primary crystallites is in the region of from 0.1 to 0.6 μm.

8. A process according to any one of Claims 1 to 7, characterised in that a catalyst is used whose primary crystallites are combined up to at least 20 % to form agglomerates of from 5 to 500 μm.

9. A process according to any one of Claims 1 to 8, characterised in that a catalyst is used, of which at least 20 % of the pores have a diameter of from 14 to 80 nm.

**Revendications**

1. Procédé de production d'oléfines inférieures de méthanol par réaction d'un mélange réactif contenant de la vapeur de méthanol et/ou de diméthyléther et de la vapeur d'eau dans un réacteur à tubes sur un catalyseur refroidi indirectement à base d'alumosilicates cristallins de type Pentasil avec un rapport atomique Si/Al d'au moins 10, caractérisé en ce que, pour la production d'un mélange oléfinique contenant au moins 5 % en poids d'éthylène, au moins 35 % en poids de propylène et au plus 30 % en poids de butylène par rapport à la teneur totale en hydrocarbures, la réaction est menée
   (a) à une pression totale de 10 à 90 kPa,
   (b) à un rapport pondéral eau/méthanol ou équivalents méthanol de 0,1 à 1,5,
   (c) à une température de l'agent de refroidissement du réacteur de 280 à 570°C,
   (d) sur un catalyseur de type Pentasil contenant des protons avec une teneur en alcalins de moins de 380 ppm, une teneur en ZnO de moins de 0,1 % en poids, une teneur en CdO de moins de 0,1 % en poids, une surface BET de 300 à 600 m²/g et un volume des pores (déterminé par porosimétrie au mercure) de 0,3 à 0,8 cm³/g.

2. Procédé selon la revendication 1, caractérisé en ce que la réaction est menée à une pression totale de 20 à 70, de préférence de 30 à 60 kPa.

3. Procédé selon la revendication 1 ou 2, caractérisé en ce que la réaction est menée à un rapport pondéral eau/méthanol ou équivalents méthanol de 0,2 à 1,0, de préférence de 0,3 à 0,8.

4. Procédé selon une des revendications 1 à 3, caractérisé en ce que la réaction est menée à une température de l'agent de refroidissement du réacteur de 300 à 570, de préférence de 350 à 500°C.

5. Procédé selon une des revendications 1 à 4, caractérisé en ce que la réaction est menée dans un réacteur à tubes à bain de sel, la température dans le catalyseur ne dépassant pas de plus de 60°C la température de l'agent de refroidissement du réacteur.

6. Procédé selon une des revendications 1 à 5, caractérisé en ce que la réaction est menée sur un catalyseur qui est composé de cristallites primaires ayant un diamètre moyen de minimum 0,1 μm et maximum 0,9 μm qui sont partiellement agglomérées, les cristallites primaires ou agglomérats étant reliés l'un à l'autre par de l'oxyde d'aluminium en fines particules.

7. Procédé selon une des revendications 1 à 6, caractérisé en ce qu'un catalyseur dont le diamètre moyen des cristallites primaires est de l'ordre de 0,1 à 0,6 μm est utilisé.

8. Procédé selon une des revendications 1 à 7 caractérisé en ce qu'un catalyseur dont les cristallites primaires sont réunies à raison d'au moins 20 % en agglomérats de 5 à 500 μm est utilisé.

9. Procédé selon une des revendications 1 à 8, caractérisé en ce qu'un catalyseur dont au moins 20 % des pores possèdent un diamètre de 14 à 80 nm est utilisé.